Europäisches Patentamt

(19) European Patent Office

Office européen des brevets

(11) Numéro de publication: **0 279 757**
A1

# (12) DEMANDE DE BREVET EUROPEEN

(21) Numéro de dépôt: 88420051.0

(22) Date de dépôt: **18.02.88**

(51) Int. Cl.⁴: **A 61 K 41/00**
A 61 K 47/00

(30) Priorité: **20.02.87 FR 8702440**

(43) Date de publication de la demande:
**24.08.88 Bulletin 88/34**

(84) Etats contractants désignés:
**AT BE CH DE ES FR GB GR IT LI LU NL SE**

(71) Demandeur: **CENTRE INTERNATIONAL DE RECHERCHE SUR LE CANCER DE L'ORGANISATION MONDIALEDE LA SANTE**
**150, Cours Albert Thomas**
**F-69372 Lyon Cedex 08 (FR)**

(72) Inventeur: **Yamasaki, Hiroshi**
**6, allée entre deux bois Bois Dieu Lissieu**
**F-69380 Lozanne (FR)**

(74) Mandataire: **Karmin, Roger et al**
**Cabinet MONNIER 150, cours Lafayette**
**F-69003 Lyon (FR)**

(54) Ensemble ou kit pour utilisation comme agent pour la destruction sélective de cellules transformées.

(57) Ensemble ou kit pour utilisation comme agent pour la destruction sélective de cellules transformées ou tumorales, caractérisé en ce qu'il comprend (a) au moins une substance toxique pour les cellules, per se ou après activation, et ne diffusant pas à travers la membrane de celles-ci et (b) au moins un moyen pour l'activation de ladite substance toxique, ainsi qu'éventuellement (c) un moyen pour la reconnaissance sélective des cellules-cibles et pour la fixation de l'ensemble (a) + (b) et éventuellement (b) sur celles-ci.

## Description

La présente invention concerne la destruction de cellules transformées ou tumorales, notamment de cellules malignes dans des organismes vivants (animaux, y compris l'homme, ou végétaux) ou hors de ces organismes. Elle concerne plus précisément la destruction sélective de telles cellules, en vue de leur élimination, ce qui la place au rang de nouvelle thérapie pour le traitement des cancers notamment.

Au surplus cette même technique pourrait être utilisée pour la localisation des sites de cellules transformées.

De nos jours, le problème de l'élimination des cellules trasformées et notamment des cellules malignes d'origine cancéreuse est traité soit par chirurgie, soit par radiothérapie, soit par chimiothérapie, soit par des traitements mixtes associant ces types de thérapies.

Des travaux menés au Centre International de Recherche sur le Cancer à Lyon (France), ont révélé que les cellules tranformées, lorsqu'elles se multiplient au sein d'une population de cellules saines ou non transformées, forment un ensemble cellulaire indépendant en ce qui concerne le phénomène de communication intercellulaire par jonction de type "gap" ; en d'autres termes, cette communication intercellulaire est effective entre cellules non transformées d'une part et entre les cellules transformées d'autre part, mais pas ou pratiquement pas entre cellules des deux catégories. Lorsque des cellules saines se transforment, elles ne peuvent plus communiquer avec les cellules normales environnantes, mais communiquent alors avec les autres cellules transformées du site (voir Enomoto T. et Yamasaki H., Cancer Res., 44, 5200, 1984).

La présente invention utilise ce dernier phénomène, dont elle constitue une application particulièrement utile et originale.

Dans le document WO-A-8 300 811, il est simplement revendiqué le développement d'un procédé de radiation destiné à l'activation de substances chimiques.

Au contraire, la présente invention concerne l'utilisation particulière de substances chimiques comportant d'une part des propriétés biologiques spécifiques et d'autre part des qualités telles qu'elles ne diffusent pas à travers la membrane des cellules. Il est absolument apparent que le document WO-A-8 300 811 ne révèle pas une telle utilisation, mais seulement la possibilité d'activer les substances chimiques, procédé qui n'est pas revendiqué dans la présente invention.

On a trouvé de manière inattendue que la destruction ou l'élimination sélective des cellules transformées présentes dans un ensemble cellulaire normalement constitué de cellules non-transformées, peut être réalisée par la combinaison de l'introduction d'une substance toxique ou potentiellement toxique appropriée et de l'activation de celle-ci dans au moins une des cellules transformées, de manière à l'amener à un état activé instable permettant la destruction des cellules transformées.

L'invention a pour premier objet un ensemble ou kit pour utilisation comme agent pour la destruction sélective de cellules transformées ou tumorales, caractérisé en ce qu'il comprend (a) au moins une substance toxique pour les cellules, per se ou après activation, et ne diffusant pas à travers la membrane de celles-ci et (b) au moins un moyen pour l'activation de ladite substance toxique, ainsi qu'éventuellement (c) un moyen pour la reconnaissance sélective des cellules-cibles et pour la fixation de l'ensemble (a) + (c) et éventuellement (b) sur celles-ci.

La substance (a) = ou ses métabolites doit donc satisfaire à deux conditions, qui sont :

- de ne pas diffuser à travers la membrane des cellules, et
- d'être toxique pour ces cellules, per se ou après activation.

En pratique, pour que la première de ces conditions soit satisfaite, il convient dans la plupart des cas que ladite substance soit préférentiellement hydrophile et ait un poids moléculaire inférieur à environ 1000 daltons ; toutefois cette caractéristique de poids moléculaire n'est pas si rigoureuse et il ne s'agit là que d'un ordre de grandeur, sachant que cette valeur limite préférée peut varier en plus ou en moins selon la configuration et/ou la taille des molécules de la substance (a) concernée. L'homme du métier sait en effet que la taille et la configuration d'une molécule ne sont pas sans incidence sur la diffusibilité de celle-ci à travers une membrane cellulaire.

Pour que la deuxième condition susdite soit satisfaite, il convient en fait que la substance (a) puisse être amenée par activation si nécessaire à un état activé instable suffisant pour tuer les cellules concernées, c'est-à-dire celles où une quantité appropriée de molécules de la substance (a) est présente. L'homme du métier est tout à fait apte à déterminer, au besoin en recourant à des essais préliminaires permettant de les sélectionner, les substances ou compositions chimiques qui peuvent convenir. Une liste non limitative des substances (a) appropriées est la suivante : diacétate de fluorescéine, dérivés de porphyrine, composés iodés, radiosensibilisateurs (par exemple dérivés hydrophiles de p-nitroacétophénone, de métronidazone, de misonidazole, etc.), Lucifer Yellow CH -colorant fluorescent disponible par exemple auprès de la société Sigma Chemical Co., Saint Louis, MO, USA ; antimétabolites monophosphates ou leurs dérivés iodés (par exemple des analogues synthétiques des nucléobases, des nucléosides et des nucléotides antimétabolites telles que 6-thioguanine, 5-fluorouracile, 6-mercaptopurine, 5-azacytidine, arabinosyl cytosine, etc.).

Le moyen pour l'activation de ladite substance peut être choisi parmi tous les moyens d'activation des substances activables connue de l'homme du métier. Il est de préférence sélectionné parmi :

- les moyens physiques ou chimiques émettant un rayonnement de nature appropriée à l'activation de la substance (a) (par exemple un rayonnement d'une longueur d'onde d'environ 400 nanomètres, des rayons lasers, des rayons X, un rayonnement UV, etc), de tels moyens étant décrits par exemple par Théodore L. Phillips "Radiation sensitizers and protectors" dans DeVita, V.T.Jr, Hellman S. Rosenberg S.A., eds, Cancer : Principles | Practice of Oncology. pp 2256-2271., chez J.B. Lippincott CO., Philadelphia, 1985.

- Les substances susceptibles de former, au contact d'une substance (a) et avec celle-ci, un ensemble activé approprié.

- les substances (a) elles-mêmes, pour autant qu'elle soient auto-activables ou rendues auto-activables, dans les conditions régnant ou en présence de substances se trouvant dans les cellules où l'activation doit être rendue opérationnelle (par exemple antimétabolites des nucléotides monophosphates).

L'ensemble ou kit destiné à permettre la destruction sélective de cellules transformées selon l'invention peut aussi comprendre, dans une variante avantageuse, un moyen (c) pour la reconnaissance sélective des cellules-cibles et pour la fixation de l'ensemble (a) + (c) et éventuellement (b) sur celles-ci. Ce moyen (c) peut être, par exemple, un virus associé à un anticorps correspondant à un antigène spécifique, dont la présence a été identifiée, par des investigations préalables, dans les cellules cibles transformées. Plus concrètement, pour un antigène dont la présence sur la membrane des cellules-cibles et dont la nature ont été identifiées, l'on met en oeuvre un ensemble anticorps-virus non pathogène conjugué avec une substance activable au sens de l'invention et l'on injecte, par voie intraveineuse ou par voie systémique.

On convient de dénommer "thérapie missile" le traitement que réalise la mise en oeuvre de tels moyens.

L'invention a également pour objet l'utilisation d'une susbtance toxique pour les cellules, per se ou après activation, et ne diffusant pas à travers la membrane de celles-ci, pour l'obtention d'un médicament destiné à la destruction sélective de cellules transformées ou tumorales.

L'invention a encore pour objet une méthode de traitement ou de diagnostic, caractérisée en ce qu'elle comprend l'utilisation in vivo ou in vitro de la combinaison de moyens constituant l'ensemble ou kit susdit.

Pour être utilisée conformément à l'objectif visé, la susbtance (a) doit être acheminée par toute technique appropriée dans au moins l'une des cellules transformées à détruire, à partir de laquelle elle passe de manière spécifique dans les autres cellules transformées du même site, par l'intermédiaire des "gap junctions". La technique préférée actuellement est la micro-injection, bien connue de l'homme du métier et décrite en détail dans l'article de Cancer Res. cité plus haut.

Dans la pratique, il apparaît avantageux de micro-injecter la substance (a) ou des solutions salines (par exemple en milieu physiologique) de la substance (a), à raison d'un volume pouvant ne pas dépasser environ 10% par rapport au volume de la cellule-cible, par des micro-injections fractionnées et espacées entre elles, par exemple à des intervalles de 10 minutes, ou par une micro-injection en continu. On a pu constater que l'injection faite dans ces conditions dans une cellule-cible de Lucifer Yellow a conduit à une destruction sélective complète d'un ensemble de 100 cellules transformées après activation à 400 nm pendant 5 à 10 minutes.

Il est préférable de respecter une attente entre la dernière micro-injection ou la fin de la micro-injection en continu et l'entrée en fonction du moyen pour l'activation de la susbtance toxique (a), afin que le passage de cette dernière dans l'ensemble des cellules transformées du site traité soit effectif. Au cas où ce passage soit insuffisant, l'on recommande de le renforcer par l'adjonction dans la solution à micro-injecter d'au moins une susbtance favorisant ce passage par "gap junction", telle que de la vitamine A, de l'AMP cyclique, des glucocorticoïdes, etc...

L'invention est maintenant illustrée en référence à l'exemple ci-aprés, qui ne la limite aucunement.

Exemple :

Cet exemple concerne la destruction de cellules transformées, par micro-injection de Lucifer Yellow CH, suivie d'une irradiation à 400 nm.

Pour la production de cellules transformées, on a utilisé des cellules BALB/c 3T3, ce système de transformation assurant la production in situ de cellules transformées sur une couche mono-cellulaire normale, et simule ainsi la cancérogenèse in vivo.

Des cellules BALB/c 3T3 ont été transfectées par un oncogène activé que l'on a isolé d'un carcinome de vessie humaire, le pEJ-ras-H ; par cette opération, on a obtenu des cellules transformées. Par micro-injection de Lucifer Yellow CH aux cellules transformées, on a pu les tuer sélectivement après irradiation à une fréquence proche de celle des rayons UV. Lorsque de nombreuses cellules sont tuées dans un foyer transformé, le foyer entier se décolle du fond de la boîte de Pétri. L'élimination de cellules se vérifie habituellement par coloration au "Trepan blue" mais peut également être confirmée par observation attentive au microscope : ces cellules ne montrent pas de capacité de prolifération ; un grand nombre d'entre elles sont décollées.

On a utilisé aussi des cellules épithéliales de foie de rat transformées et non transformées. Puisqu'à l'heure actuelle, il n'existe pas de méthode d'induction in situ de foyer épithélial transformée par des cancérogènes, on a cocultivé articifiellement des cellules épithéliales de foie de rat transformées et non transformées, de sorte que les deux types de cellules puissent être en contact. Lorsque les cellules transformées (IAR 27 ou IAR 6-1) sont cultivées avec des cellules non transformées (IAR 20), la frontière entre les deux types de cellules est clairement visible à cause de la différence de leur aspect morphologique. Par micro-injection de Lucifer Yellow CH, on a pu constater une diffusion sélective du colorant dans les cellules du même type, et après exposition à un rayonnement proche de celui des UV, la mort des cellules-cibles micro-injectées.

L'efficacité de l'élimination sélective dépend directement de la capacité de communication des cellules-cibles. Lorsque plus de cellules étaient atteintes par le Lucifer Yellow CH on constatait, après irradiation par lumière bleue, qu'un plus grand nombre de cellules étaient tuées. Après avoir procédé à une micro-injection de Lucifer Yellow CH dans une seule cellule BALB/c 3T3, on constatait presque toujours la fluorescence du colorant dans les 25 à 35 cellules qui l'entouraient alors que les cellules épithéliales du foie de rat présentaient une capacité de communication plus élevée (50 à 100 cellules) ; ainsi le nombre de cellules tuées par injection est plus élevé dans les cellules du foie de rat.

L'efficacité d'élimination a également été augmentée par stimulation de la communication des "gap junctions" par un composé exogène : après addition de dibutyryl c-AMP (1mM) dans une boîte de Pétri qui contient des foyers transformés de cellules BALB/c 3T3, on a observé que les communications intercellulaires sont stimulées dans les foyers transformés et dans la monocouche non transformée ; cependant, les deux types de cellules ne communiquent pas entre eux. On constate que l'élimination sélective se faisant plus facilement dans les cellules transformées. Il est également important de noter qu'un traitement prolongé au c-AMP provoquait la suppression de la spécificité de communication intercellulaire, les phénotypes transformés ont alors commencé à disparaître.

Les résultats présentés ci-dessus montrent que les cellules transformées peuvent être tuées avec succès par micro-injection de substances cytotoxiques ou potentiellement cytotoxiques ; une injection unique peut tuer un grand nombre de cellules voisines car les "gap junctions" peuvent concourir à faire diffuser la susbtance.

Les cellules normales voisines des cellules micro-injectées ne subissent pas d'effet secondaire ; la compartimentation de la communication sélective protège les cellules normales. Etant donné les problèmes d'effets secondaires couramment rencontrés avec un grand nombre d'agents chimiothérapeutiques, on pourrait les éviter en utilisant cette méthode pour la thérapie anti-cancéreuse.

Sans vouloir être lié par une quelconque théorie, l'on peut émettre l'hypothèse que :

- les cellules transformées d'un site communiquent entre elles, mais pas avec les cellules normales qui les environnent, cette communication se faisant par le biais d'une structure membranaire appropriée acquise, c'est-à-dire des jonctions de type "gap" (communément dénommées "gap junctions") ;

- le passage, naturel ou activé, d'une substance (toxique pour les cellules après activation et choisie de telle sorte qu'elle ne diffuse pas à travers la membrane de celles-ci) atteint finalement l'ensemble des cellules transformées d'un site où cette substance a été introduite, et pas ou pratiquement pas les cellules non transformées environnantes ;

- la substance toxique, une fois à un état activé instable, se désactive spontanément et émet une énergie suffisante pour tuer les cellules le contenant.

## Revendications

1. Ensemble ou kit pour utilisation comme agent pour la destruction sélective de cellules transformées ou tumorales, caractérisé en ce qu'il comprend (a) au moins une substance toxique pour les cellules, per se ou après activation, et ne diffusant pas à travers la membrane de celles-ci et (b) au moins un moyen pour l'activation de ladite susbtance toxique, ainsi qu'éventuellement (c) un moyen pour la reconnaissance sélective des cellules-cibles et pour la fixation de l'ensemble (a) + (c) et éventuellement (b) sur celles-ci.

2. Ensemble ou kit selon la revendication 1, caractérisé en ce que la substance toxique (a) est hydrophile et a un poids moléculaire inférieur à environ 1000 daltons.

3. Ensemble ou kit selon la revendication 2, caractérisé en ce que la substance toxique (a) est choisie parmi : le diacétate de fluorescéine, les dérivés de porphyrine, les composés iodés, les radiosensibilisateurs, le Lucifer Yellow CH, et les antimétabolites monophosphates ou leurs dérivés iodés.

4. Ensemble ou kit selon la revendication 1, caractérisé en ce que la substance toxique pour les cellules et ne diffusant pas à travers la membrane de celles-ci est un métabolite de la substance toxique (a).

5. Ensemble ou kit selon la revendication 1, caractérisé en ce que le moyen pour l'activation de la substance toxique (a) est choisi parmi les moyens physiques ou chimiques émettant un rayonnement, les substances formant avec la susbtance (a) un ensemble activé, et les substances (a) elles-mêmes pour autant qu'elles soient ou soient rendues auto-activables.

6. Ensemble ou kit selon la revendication 1, caractérisé en ce qu'il comprend un moyen (c) composé d'un virus associé à un anticorps correspondant à un antigène spécifique présent dans les cellules-cibles transformées.

7. Utilisation d'une substance toxique pour les cellules et ne diffusant pas à travers la membrane de celles-ci, pour l'obtention d'un médicament destiné à la destruction sélective de cellules transformées ou tumorales.

8. Utilisation selon la revendication 7, caractérisée en ce que la substance toxique est hydrophile et a un poids moléculaire inférieur à environ 1000 daltons.

9. Utilisation selon la revendication 7, caractérisée en ce que la substance toxique est choisie parmi le diacétate de fluorescéine, les dérivés de porphyrine, les composés iodés, les radiosensibilisateurs, le Lucifer Yellow CH, et les antimétabolites monophosphates ou leurs dérivés iodés.

10. Méthode de traitement ou de diagnostic, caractérisée en ce qu'elle comprend l'utilisation in vitro d'un ensemble ou kit selon l'une quelconque des revendications 1 à 6.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,D | WO-A-8 300 811  (QUENTRON OPTICS) <br> * Page 1, ligne 1 - page 3, ligne 7; revendications 1,5-8,11 * | 7-9 | A 61 K  41/00 <br> A 61 K  47/00 |
| A |  | 1-6,10 | |
| | --- | | |
| A | EP-A-0 175 617  (CYTOGEN CORP.) <br> * Page 27, lignes 22-30; page 39, ligne 24 - page 43, ligne 18; revendications 9,15,38,39 * | | |
| | ----- | | |

**DOMAINES TECHNIQUES RECHERCHES (Int. Cl.4)**

A 61 K

Le présent rapport a été établi pour toutes les revendications

| Lieu de la recherche | Date d'achèvement de la recherche | Examinateur |
|---|---|---|
| LA HAYE | 04-05-1988 | BENZ K.F. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

 

& : membre de la même famille, document correspondant

EPO FORM 1503 03.82 (P0402)